# EUROPEAN PATENT SPECIFICATION

(11) **EP 0 885 615 B1**
(45) Date of publication and mention of the grant of the patent: **26.03.2003**
(21) Application number: 98116817.2
(22) Date of filing: 04.07.1994
(51) Int. Cl.: A61K 49/00

(54) **Protein encapsulated insoluble gas microsperes and their preparation and use as ultrasonic imaging agents**
Proteinverkapselte, unlösliche, gasenthaltende Mikrokügelchen, deren Herstellung und Anwendung als bildgebende Mittel für Ultraschall
Microsphères à gaz insolubles, encapsulées par une protéine, leur préparation et application comme produits pour imagerie par ultrason

(30) Priority: 02.07.1993 US 86717; 26.01.1994 US 187656
(43) Date of publication of application: 23.12.1998
(62) Divisional of application: 94304902.3
(73) Proprietor: MOLECULAR BIOSYSTEMS, INC., San Diego California 92121 (US)
(72) Inventor: Lambert, Karel J., San Diego, California 92126 (US); Podell, Sheila Benay, San Diego, California 92122 (US); Jablonski, Edward G., Escondido, California 92029 (US); Hulle, Carl, San Diego, California 92122 (US); Hamilton, Kenneth, Cardiff, California 9200 (US); Lohrmann, Rolf, La Jolla, California 92037 (US)
(74) Representative: Wright, Simon Mark

(56) References cited:
- EP-A- 0 224 934
- EP-A- 0 324 938
- EP-A- 0 359 246
- WO-A-92/05806
- WO-A-93/05819

## Description

### Technical Field

This invention relates to methods for the production of ultrasonic imaging agents composed of proteinaceous microspheres encapsulating gases.

### Background

Diagnostic ultrasonic imaging is based on the principle that waves of sound energy can be focused upon an area of interest and reflected in such a way as to produce an image thereof. An ultrasonic scanner is placed on a body surface overlying the area to be imaged, and ultrasonic energy in the form of sound waves are directed toward that area. The scanner detects reflected sound waves and translates the data into video images. When ultrasonic energy is transmitted through a substance, the amount of energy reflected depends upon the velocity of the transmission and the acoustic properties of the substance. Changes in the substance's acoustic properties (e.g., variations in acoustic impedance) are most prominent at the interfaces of different acoustic densities, such as liquid-solid or liquid-gas. Consequently, when ultrasonic energy is directed through tissue, organ structures generate sound reflection signals for detection by the ultrasonic scanner. These signals can be intensified by the proper use of a contrast agent.

Ultrasound imaging agents of particular importance employ the use of gas because of its efficiency as a reflector of ultrasound. Resonant gas bubbles scatter sound a thousand times more efficiently than a solid particle of the same size. Ophir and Parker describe two types of gas-containing imaging agents as being: (1) free air bubbles and (2) encapsulated air bubbles (Ultrasound in Medicine and Biology 15(4):319-333, 1989). However, free gas bubbles of the appropriate size are too short-lived to be effective for most *in vivo* applications (Meltzer, et al., Ultrasound in Medicine and Biology 6:263-269, 1980). Ophir and Parker point out that the development of encapsulated gas bubbles was an attempt to overcome this problem.

The second major class of gas-containing ultrasound contrast agents described by Ophir and Parker are the encapsulated microbubbles, hereinafter referred to as "microspheres". The gas bubble is surrounded by a shell composed of a protein or other biocompatible material. A current commercial microsphere contrast agent is ALBUNEX® (Molecular Biosystems, Inc., San Diego, CA) which is composed of human serum albumin encapsulated air microspheres. See U.S. Patent Nos. 4,572,203 and 4,844,882.

Air microspheres have been shown to quickly lose echogenicity when subjected to pressures of 150 mm Hg, such as would be encountered during injection and circulation *in vivo* (deJong, N. et al., Ultrasound Med. Biol. 19 : 279-288, 1993). Present encapsulating technology has yet to produce a material suitable as an ultrasound contrast agent that will survive long enough *in vivo* for most desired applications. In fact, an agent capable of imaging the myocardial wall must withstand transient pressures pulses of at least 250 mm Hg (about 5 psig).

In an effort to solve the pressure-instability problem of microspheres, recent teachings have centered on improving the shell, because, it is believed, the microsphere shells or "membranes" are too fragile or brittle under pressure, resulting in rapid collapse *in vivo*. Giddey (PCT/EP91/01706; PCT 92/05806) stated, "because of their rigidity, the membranes cannot sustain sudden pressure variations to which the microspheres can be subjected, for instance during travel through the bloodstream, these variations or pressure being due to heart pulsations." To overcome shell rigidity, he proposed to pre-emulsify air in a protein solution containing a large percentage of a viscosifying agent (40%-80% polyols) and subject it to mechanical shear in a high speed blender. Bubbles of the appropriate size are collected and coated with a suitable surfactant to stabilize them in a soft shell.

Holmes (PCT WO 92/17213) proposed to enhance the *in vivo* stability of protein microspheres by strengthening the shell with biodegradable chemical crosslinking reagents.

Bichon et al. (EPA 90/810367) and Schneider et al. (Inv. Radiol. 27 : 134-139, 1992) describe the production of porous (5 to 2000 nm pore size) polymeric "microballoons". They report in the European Patent Application that "the microporous structure of the microballoon's envelope is a factor of resiliency, i.e., the microspheres can readily accept pressure variation without breaking."

Erbel and Zotz (U.S. Patent No. 5,190,982) describe a cross-linked polymeric microcapsule in which air is entrapped.

Schneider et al. (EPA 554,213) indicates that the pressure resistance of microspheres can be improved by having at least a portion of the gas that is encapsulated be a gas which has a S_{gas}/MW_{gas} ≤ 0.0031, where S_{gas} is the water solubility of the gas in liters/liter and MW_{gas} is the average molecular weight of the gas in daltons. Table 1 of the reference lists N₂, SF₆, CBrF₃ and CF₄ as meeting this criterion. The reference teaches that these microspheres may be made in either of two ways. The first is a two-step method in which air-containing microspheres are prepared by a known method and the air is replaced with the insoluble gas by a gas-exchange method, e.g., by incubating the air-filled microspheres under an atmosphere of the insoluble gas for an adequate time.

The second is a one-step method in which microspheres are made by the method of EPA 324,938 (See Example 1 of the reference) using an insoluble gas instead of air. In that method, the gas was passed over a solution of a shell-forming material (e.g., a solution of albumin) while a sonicator horn was lowered into the vessel and then removed.

Unfortunately, neither of these methods is practically useful for making stable suspensions of protein encapsulated insoluble gas-filled microspheres. Using the first (two-step) method, only a small number of air-filled albumin microspheres can survive the exposure to an insoluble gas environment (the second step of the two-step method). The efflux of soluble gases (air) out of the microspheres is greater than the influx of insoluble gas into the microspheres which results in complete collapse of the microspheres, leaving only shell debris. This effect is particularly pronounced with the more insoluble gases such as perfluoroethane. The second process produces microspheres which lose volume as pressure is applied and do not exhibit recovery after the release of pressure. It is believed that both of these processes produce inferior microspheres because the microspheres contain significant amounts of air and that the presence of air during formation may lessen the benefits of producing the microspheres in the presence of insoluble gas alone. It is noted, in this regard, that prior investigators have considered the presence of oxygen essential in making microspheres by cavitation.

Suslick reported that ultrasound-associated cavitation was suitable as a method of production of microspheres only in the presence of oxygen. In detailed studies, Suslick et al., (Proc. Natl. Acad. Sci. 88:7708-7710, 1991; J. Am. Chem. Soc. 112:7807-7809, 1990) reported that oxygen participates in the cavitation-induced intermolecular rearrangement of disulfide bonds needed for a stable protein shell. Suslick states "We find that microcapsule formation is strongly inhibited by the absence of 0₂". He goes on to state, "If the reaction is run under an inert atmosphere (He, Ar or N₂), microcapsules are not formed." "Experimentally, high concentrations of microbubbles are synthesized only when the reaction is run under 0₂ or air." See also, U.S. 4,774,958. The previous belief that air was necessary for the formation of albumin microspheres was also noted in Holmes (PCT WO 92/17213). Therein, the production of microspheres containing various low molecular weight gases was disclosed. However, when describing the production of albumin microspheres by sonication, the authors stated, "Another well-established method described, i.e., US-A-4,774,958, for creating a gas-containing bubble is by sonication of the mixture in the presence of air." (Emphasis added.)

It has now been found that high concentrations of proteinaceous microspheres, entrapping relatively insoluble gas, can be made by ultrasound or mechanical cavitational processes in the presence of the insoluble gas without the presence of oxygen. Such microspheres exhibit surprising and greatly improved stability and elasticity to applied pressure, with better or equivalent echogenicity. The proteinaceous shell prevents coalescence and resists expansion due to diffusion of dissolved atmospheric gases from the surrounding environment.

The current invention relates to a novel procedure for the production of protein-shelled microspheres which employs mechanical energy in the form of shear forces. These forces are responsible for mechanically shearing a liquid-gas mixture to form a microbubble suspension, and also for causing hydrodynamic cavitation which releases energy. This energy can be absorbed by the surrounding liquid to effect localized protein denaturation and deposition at a gas-liquid interface to form discrete microspheres. Hydrodynamic cavitation can be distinguished from ultrasonic (acoustic) cavitation on the basis of the way in which each produces pressure variations on liquid systems which lead to the release of energy. In the former, pressure variations are produced by the rapid flow of a liquid through an orifice or across a surface, whereas in the latter, cycles of high frequency sound waves produce rapid local pressure variations. (See F. Ron Young. 1989 Cavitation Pages 4-5, McGraw-Hill Book Co. London). Additionally, hydrodynamic cavitation is produced in a flowing liquid, i.e., a liquid which is flowing through or across a stationary object. In comparison, acoustic cavitation is produced in a liquid system which must remain stationary through enough cycles of increasing and decreasing pressure (positive and suction pressure) to exhibit cavitation. Even in a continuous flow sonication system such as that described in U.S. Patent No. 4,957,656, the residence time in an acoustic cavitation process makes it harder to control than in a true single-pass hydrodynamic cavitation system such as is described by the present invention.

Microbubble suspensions produced by mechanical shear forces are used per se as contrast agents or formed into microspheres by further processing. For instance, PCT Publication No. WO 92/05806 describes the preparation of a microbubble suspension, which they refer to as a "foam", of a filmogenic protein which is prepared by whipping a protein solution containing viscosifiers into a coarse foam at a constant temperature below that which would denature the protein. The resultant foam is then sheared mechanically to form bubbles of a desired range which are stabilized by the presence of the viscosifiers. The bubbles may then be further processed into microspheres by heat denaturation or by the addition of cross-linkers to harden the protein film surrounding the bubbles.

European Patent Application Pub. No. 0 450 745 A1 describes a process for making microspheres by forming an oil-in-water emulsion by mechanical shearing and simultaneously or subsequently adding a water-insoluble polymer which deposits at the interface. The hydrophobic phase is then evaporated to form air or gas filled microspheres.

Thus, the present invention also relates to an improved method for making microspheres from heat-denaturable protein by subjecting a protein solution to mechanical shear forces. Such forces create a suspension of microbubbles that are simultaneously or subsequently encapsulated by a discrete shell. Because of the nature of cavitational heating, the denaturation of the protein is localized and forms the shell by depositing at the liquid-gas interface. This new method is easier to scale-up and leads to improved product yields as compared to the prior acoustic-based methods of making microspheres.

### Disclosure of the Invention

The present invention provides a method of making encapsulated gas microspheres useful as an ultrasonic imaging agent comprising:
a) providing an aqueous solution of a heat-denaturable protein at a temperature necessary to achieve incipient denaturation temperature during subsequent mechanical emulsification;
b) combining the solution with a gas;
c) emulsifying the protein solution and gas mixture by mechanically shearing the mixture to form a suspension of gas microbubbles having a mean diameter in the range of about 0.1 to about 10 microns; and
d) encapsulating the gas microbubbles to form microspheres by mechanically cavitating the suspension to cause the protein to become denatured and thereby deposited at the gas-solution interface.

### Brief Description of the Drawings

Fig. 1 is an exploded schematic view of one type of mill (a Gaulin mill) that may be used in the mechanical cavitation process of the invention;
Fig. 2 is an exploded schematic view of another type of mill (a Bematek mill) that may be used in the mechanical cavitation process of the invention;
Fig. 3 is an exploded schematic view of still another type of mill (a Silverson mill) that may be used in the mechanical cavitation process of the invention.
Fig. 4a shows the pressure resistance of air-filled albumin microspheres. A microsphere suspension was placed in a syringe and pressurized at 40 psig. Particle distributions before and after pressurization are shown.
Fig. 4b shows the pressure resistance of perfluoropropane-filled albumin microspheres. A microsphere suspension was placed in a syringe and pressurized at 40 psig. Particle distributions before and after pressurization are shown.
Fig. 4c shows the pressure resistance of perfluoroethane-filled albumin microspheres. A microsphere suspension was placed in a syringe and pressurized at 40 psig (2.76 bar). Particle distributions before and after pressurization are shown.
Fig. 4d shows the pressure resistance of sulfur hexafluoride-filled albumin microspheres. A microsphere suspension was placed in a syringe and pressurized at 40 psig (2.76 bar). Particle distributions before and after pressurization are shown.
Fig. 4e shows the pressure resistance of argon-filled albumin microspheres. A microsphere suspension was placed in a syringe and pressurized at 40 psig (2.76 bar). Particle distributions before and after pressurization are shown.
Fig. 5 shows the pressure resistance of dilute suspensions of microspheres at 3.0 psig (0.21 bar). A diluted suspension of microspheres were placed in a 1 cm cuvette and subjected to 3.0 psig (0.21 bar) at the time t=30 seconds. Shown are data for perfluoroethane, perfluoropropane, sulfur hexafluoride and air microspheres.
Fig. 6 shows pressure resistance of dilute suspension of argon microspheres at 3.0 psig (0.21 bar). Diluted argon microspheres were placed in a 1 cm cuvette and subjected to 3.0 psig (0.21 bar) at time t=30 seconds.
Fig. 7 shows the effect of degassed buffer on microspheres. Microspheres were added to increasing amounts of degassed buffer, with mixing, and the mixture brought to constant volume for determination of concentration. Data for air, perfluoropropane, perfluoroethane, and sulfur hexafluoride microspheres are plotted versus volume of degassed buffer.
Fig. 8 shows graphs of data described in Example 11, *infra*.

### Modes for Carrying Out the Invention

Microspheres may be formed in an aqueous suspension from the mechanical cavitation of aqueous solutions of filmogenic proteins in the presence of an insoluble gas and in the substantial absence of oxygen, i.e., under anaerobic (closed system) conditions. The microspheres are echo reflective and of a size suitable for transpulmonary passage, with a mean diameter less than 10 microns and greater than 0.1 microns. The size distribution may be altered by fractionation into larger or smaller microsphere populations. The microspheres may or may not be concentrated by removal of excess aqueous phase, or collected and resuspended in a second aqueous solution.

The gas used to make these microspheres need only be pharmacologically acceptable and preferably insoluble in the aqueous media in which they are placed (i.e., initially the medium in which they are made and, when used, in the blood). Solubility in water is a close approximation of solubility in such media. The term "gas" refers to any compound which is a gas or capable of forming gas at the temperature at which imaging is being performed (typically normal physiological temperature). The gas may be composed of a single compound or a mixture of compounds. Appropriate gases would include, but are not limited to, fluorine-containing gases such as sulfur hexafluoride, perfluoroethane, perfluoropropane, perfluoromethane, and perfluorobutane. Solubility of a gas can be defined by determining the Bunsen Coefficient of the gas of interest. This value is the volume of gas which is absorbed by a unit volume of solvent. (see Wen, W-Y, Muccitelli, JA, J. Sol. Chem. 8:225-240 (1979)) Gas suitable for use in the present invention should preferably have a Bunsen Coefficient in water at 25°C of less than 0.01 mL/mL of solution. Table 1 gives the Bunsen Coefficients of several gases.

**Table 1**

| BUNSEN COEFFICIENTS OF GASES IN WATER (1 atmosphere, mL/mL) | | |
|---|---|---|
| **Gas** | **5°C** | **25°C** |
| Carbon Dioxide | 1.383 | 0.824 |
| Argon | 0.047 | 0.033 |
| Oxygen | 0.043 | 0.031 |
| Nitrogen | 0.021 | 0.016 |
| Sulfur hexafluoride | 0.008 | 0.0054 |
| Perfluoromethane | 0.0082 | 0.00504 |
| Perfluoroethane | 0.0027 | 0.00138 |
| Perfluoropropane | 0.0016 | N/A |
| Perfluorobutane | 0.0007 | N/A |

Another characteristic of the gas contained in the microspheres is that the diffusivity of the gas is preferably less than 4 x 10⁻⁵ cm²/sec at 25°C in water. It should be noted, however, that the diffusivity constant varies in different solvents and at different temperatures, but for purposes of selecting a gas, the gas should meet this criterion.

Pharmacologically acceptable refers to the property that the selected gas must be biocompatible and have minimal toxicity.

Perfluoropropane is preferred because it provides an insoluble gas that (1) will not condense at the temperatures of manufacture and use, (2) has no isomeric forms, (3) produces microspheres that exhibit excellent pressure resistance, and (4) is pharmacologically acceptable.

The gas microbubble is encapsulated by a filmogenic protein shell. The term filmogenic refers to the ability of the protein to form a shell around the entrapped gas, with hydrophilic groups oriented externally and hydrophobic groups oriented internally, upon protein insolubilization (caused by heat denaturation). The protein will necessarily have both hydrophilic and hydrophobic amino acids. Suitable proteins will include naturally occurring proteins such albumin, gamma-globulin (human), apo-transferrin (human), b-lactoglobulin, and urease. Although naturally occurring proteins are preferred synthetic proteins (homopolymeric or heteropolymeric) which exhibit tertiary structure and are susceptible to heat denaturation may be used. Particularly well suited for the present invention is albumin, and more particularly, human albumin. The protein is present in the solution at a concentration in the range of about 0.1 to 10% w/v, preferably about 1 to 5% w/v, and most preferably about 1% w/v.

Proteins suitable use in for the present invention, or the resulting microspheres, may be chemically modified for the purpose of organ targeting or quenching immunogenic activity (e.g., modification with polyethylene glycol). However, the present invention does not involve addition of chemical crosslinking agents, or additional modifications to the proteins for the purpose of forming the microspheres.

The microspheres are formed by insolubilizing portions of a protein in solution as a result of cavitation in the presence of a gas, preferably without the presence of oxygen (i.e., in a closed system in which air contamination is avoided.) Such protein insolubilization is characterized primarily by local protein denaturation and orientation around the gas core, the latter of which may be enhanced in the presence of the insoluble gas.

The system used to heat-insolubilize the protein for formation of the microspheres of the present invention is anaerobic, i.e., closed to the atmosphere, and is referred to as a "closed system". In comparison, an "open system" is one which is open to the atmosphere. The gas entrapped in microspheres made in such a closed system will necessarily contain only the gas used for formation. Contamination by atmospheric gases can be monitored using an O₂ electrode to measure the presence of O₂ in the system effluent. In the present invention, microspheres are produced which initially contain only the gas used for formation. However, when the gas content is determined experimentally, there is a certain amount of unavoidable contamination by the atmospheric gases during the experimental procedure, thus causing the amount of resultant gas measured to be less than 100%. Accordingly, measurements of gas greater than 85% are representative of microspheres whose initial gas content was entirely insoluble gas.

After formation of the microspheres, exposure to the atmosphere should be avoided during packaging. For example, microspheres should be sealed in vials or other air-tight containers within 5 to 30 seconds from the time they exit from the closed system. Additionally, any head space in the vials should be removed and replaced with the gas used in formation during packaging.

Filled with an insoluble gas, these protein microspheres exhibit remarkable stability, surviving exposure of 40 psig (>2000 mm Hg, 2.76 bar) at a concentration of about 1.0 x 10⁹ microspheres per mL. The microspheres also exhibit elasticity in a dilute suspension, exhibiting compression under pressure of 3-10 psig (0.21 to 0.69 bar), and returning to the original volume upon the release of pressure. Additional chemical cross-linking would be disadvantageous because the resultant microspheres would have too rigid a structure to exhibit enhanced pressure stability.

The microspheres, unlike free microbubbles, are resistant to coalescence and diffusion driven expansion. Microspheres containing insoluble gas incubated in air or oxygen saturated solution, at various temperatures, do not increase in mean diameter or increase in total volume. The protein shell, although elastic when subjected to pressure, is strong enough to resist expansion or tearing by gas diffusion or exchange. The presence of the protein shell prevents coalescence, and maintains the gas in small, individual bubbles for up to several months, similar to air-filled protein microspheres. The inability of insoluble gas-filled microspheres to measurably expand by exchange with solvated atmospheric gases is a novel and key property for the use of this material as an ultrasound agent.

Insoluble gas-entrapped protein microspheres exhibit resistance to collapse upon exposure to degassed aqueous solutions. Unlike free microbubbles or encapsulated microspheres filled with air, insoluble gas-filled microspheres may be added to vacuum degassed water and maintain integrity at high dilution. Air-filled material collapses in blood due to the efflux of the oxygen component of the gas phase. The ability of microspheres filled with an insoluble gas to resist collapse in a partially degassed or pressurized environment increases dramatically the duration of ultrasound contrast *in vivo*.

The microspheres are produced by mechanical cavitation. A process of ultrasound production of air-filled microspheres has been described by Cerny (USP 4,957,656).

Mechanical cavitation is used in the present invention for making the insoluble gas-filled microspheres of this invention. It may also be used to make air-filled or soluble gas (e.g., N₂, H₂, argon)-filled microspheres.

In the novel mechanical cavitation procedure of the present invention, the aqueous solution of heat-denaturable protein is provided at a temperature necessary to achieve incipient denaturation temperature during the subsequent mechanical emulsification of the solution. The denaturation temperature of the protein in solution will normally be in the range of 50 to 100°C. It can be obtained from tables of thermal protein denaturation in the literature, or experimentally by any known method. For example, to determine the denaturation temperature experimentally, a protein solution can be heated in a water bath while stirring. The denaturation temperature is the temperature at which insoluble material is first observed. Note that the denaturation temperature is affected by the nature, purity and source of the protein, the concentration of protein in the solution, the pH, buffer, ionic strength, the presence of stabilizers and the presence of chemical denaturants or detergents. Therefore, it is necessary to determine the denaturation temperature of the protein in the environment in which it will be employed to make microspheres. If desired, additives such as detergents or polar solvents can be employed to change the temperature at which denaturation takes place.

Table 2 gives the denaturation temperatures of several naturally occurring proteins which were determined experimentally as described above:

**Table 2**

| **PROTEIN** | **CONCENTRATION** | **pH** | **SOLVENT** | **T**_{**denaturation**} |
|---|---|---|---|---|
| Human Serum Albumin, USP Swiss Red Cross (Bern. Switzerland) | 50 mg/mL | 6.9 | 0.9% NaCl, 4mM Sodium Caprylate, 4mM Tryptophanate | 75°C |
| Human Serum Albumin, USP Swiss Red Cross (Bern. Switzerland | 10 mg/mL | 6.9 | 0.9% NaCl, 1 mM Sodium Caprylate, 1 mM Tryptophanate | 78°C |
| β-Lactoglobulin, Sigma (St. Louis, MO) | 25 mg/mL | 7.6 | USP Water | 90°C |
| αβ-Globin, Sigma (St. Louis, MO) | 25 mg/mL | 5.0 | USP Water | 90°C |
| Lysozyme Sigma (St. Louis, MO) | 100 mg/mL | 7.5 | 5 mM TRIS*, 2mM DTT*** | 31°C as determined immediately after addition of DTT |
| Human Gamma Globulin, acid pH method, Sigma (St. Louis, MO) | 40 mg/mL | 5.0 | 10 mM MES**, pH 5.0 | 66°C |
| Human Gamma Globulin, alkaline pH method, Sigma (St. Louis, MO) | 40 mg/mL | 9.8 | 10 mM TRIS, pH 9.8 | 69°C |
| apo-Transferrin, Sigma (St. Louis, MO) | 20 mg/mL | 7.5 | 10 mM TRIS* | 71°C |

| | | | | |
|---|---|---|---|---|
| * TRIS = 2-amino-2-(hydroxymethyl)-1,3-propanediol | | | | |
| ** MES = 2-(N-morpholino)ethanesulfonic acid | | | | |
| *** DTT = dithiothreitol | | | | |

Each apparatus employed to shear the protein solution/gas mixture will cause a certain amount of additional heating of the protein solution due to the mechanical shear forces exerted on the solution. That heat must be sufficient to cause localized denaturation of the protein at the gas-liquid interface. It is thus important to determine the amount of temperature increase caused by the apparatus so that the temperature at which the protein solution is introduced into the apparatus can be adjusted to achieve such local thermal denaturation. Specifically, the bulk temperature of the liquid in the apparatus must coincide with the incipient denaturation temperature immediately prior to cavitation. The cavitation event generates the additional heat necessary to locally denature the protein. Incipient denaturation temperature is defined as the temperature at which the protein is on the verge of denaturation, but the solution does not contain any denatured protein. This temperature is just below, typically 1 to 5°C below, the denaturation temperature. If necessary, the starting protein solution may be preheated prior to being introduced into the apparatus to a temperature that allows the incipient denaturation temperature to be reached.

Once the proper starting temperature of the protein solution has been achieved, the solution is combined with a suitable gas, for example by introducing the gas into the protein solution prior to or during the emulsification step at a volume to volume ratio in the range of about 5% to 200% gas:liquid, preferably about 20% to 100%. The proper gas:liquid ratio will depend on the geometry of the apparatus, the physical characteristics of the gas (solubility, density, molecular weight, etc.), and can be adjusted to optimize output.

After the gas and protein solution are combined, the mixture is emulsified and subjected to cavitation under conditions that produce microspheres. This is accomplished using an apparatus in which mechanical shearing and hydrodynamic cavitation can be produced, such as high speed mixers, mills, fluidizers and the like. A preferred apparatus is a colloid mill which is defined as "a machine consisting of a high-speed rotor and a stator, dispersion or emulsification being effected by the opposing faces." Advanced Filtration and Separation Technology, p.108-110. Examples of specific milling apparatuses which can be used are as follows:

| | |
|---|---|
| Model #2 1/2 | Bematek, Beverly, MA |
| Model W250V | Greerco, Hudson, NH |
| Model 2F | APV Gaulin, Everett, MA |
| Model L4R | Silverson, Chesham, UK |
| Model Polytron PT3000 | Kinematica, Littaw, Switzerland |

When used to make insoluble gas-filled microspheres, the colloid mills should be closed to the atmosphere so as to avoid introducing air into the mixture.

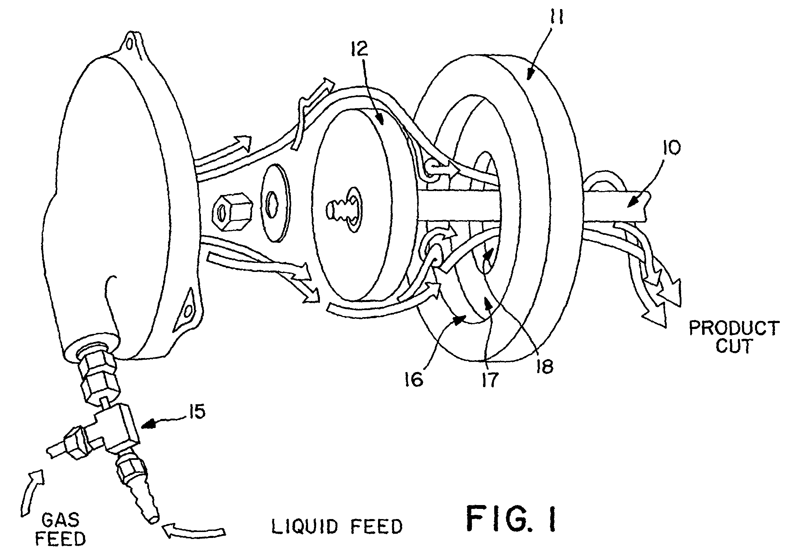

Figures 1-3 provide further details of several types of mills that may be used in the mechanical cavitation process.

Fig. 1 depicts the essential elements of a Gaulin mill. These are: a rotating shaft (10) operably connected to a motor (not shown); a disc rotor (12) affixed to the end of the shaft (10); and a stator (11). Stator (11) has a central bore opening (18) and a counterbore (16) into which the rotor is received. In this mill the protein solution and gas are fed through the mill via a "tee" (15). The protein solution/gas mixture is emulsified and cavitates between the surfaces of the rotor and stator. The "gap" in this mill is the space between radial surface (17) of the stator counterbore and the circumferential radial surface of the rotor. The temperature of the microsphere product will be taken (e.g. with a thermocouple, not shown) as the mixture exits past the stator (11).

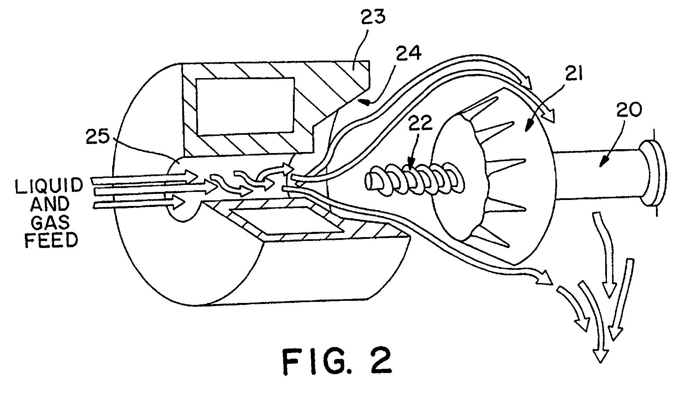

Fig. 2 shows the essential elements of a Bematek mill. This mill is similar in structure and function to the Gaulin mill of Fig. 1 -- the main difference being the configurations of the rotor and the stator counterbore. It includes a rotary shaft (20) that carries a frustroconical rotor (21) that has a threaded leading end (22) and a stator (23) that has a central cylindrical opening (25) and a frustroconical counterbore (24) that is adapted to receive the rotor. The protein solution/gas mixture is fed into this mill via opening (25). The gas and solution are mixed as they pass by the threads on the shaft (22) and the mixture is emulsified and subjected to cavitation as it passes through the gap of the mill. The gap is defined by the space between the conical surfaces of the rotor and stator.

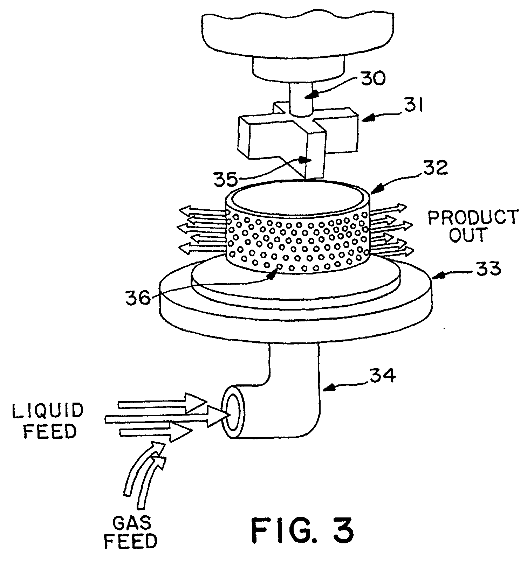

Fig. 3 illustrates a Silverson mill. The structure of this mill is quite different from those of the mills of Figs 1 and 2. The depicted Silverson mill has a rotating shaft (30) that carries a paddle blade rotor (31). The rotor is received in a cup-shaped perforated screen stator (32). The stator is mounted on a housing (33) fitted with an inlet fitting (34). The inlet fitting (34) extends up into the housing (33) opening at the bottom center of the perforated screen stator (32). The housing has a central opening (not shown) that communicates with the inlet fitting and with an opening (also not shown) in the bottom of the stator. In this mill the solution/gas is fed via the inlet fitting into the bottom of the stator and is emulsified and cavitated between the flat faces (35) of the paddle rotor and the inner cylindrical surface of the stator. The "gap" of this mill can be defined as the space between the rotor (31) and stator (32), but the effect of the gap size on the process is influenced by the size of the perforations (36) of the stator.

After passing through the mill the product may be cooled, typically to 10 - 20°C, and defoamed by settling or by adding biocompatible defoaming agents that do not adversely affect the microspheres.

Passing the mixture through such a mill or equivalent device emulsifies and cavitates the mixture to form microspheres in the range of about 0.1 to 10 microns (mean diameter). Microsphere size may be determined by a suitable particle counter, for example a Coulter Multisizer II (Coulter Electronics, Hialeah, Fl).

When using a mill such as those described in Figs. 1-3, the rotor speed, gap size and gas:liquid ratio are the principal process parameters which affect the characteristics (mean size, size distribution, and concentration of microspheres) of the microsphere product. Those parameters are adjusted empirically to provide a product having the desired characteristics. For any given product, its characteristics are defined clinically. For instance, putative specifications for perfluoropropane microspheres used for myocardial perfusions are: mean size, 4 microns; size distribution, 90% under 10 microns; concentration, 7 x 10⁸ to 2 x 10⁹ microspheres/mL.

The invention is further illustrated by the following examples. These examples are not intended to limit the invention in any manner.

### EXAMPLE 1

### Mechanical Cavitation Process Temperature Monitoring and Control for Human Serum Albumin

As described above, the protein solution is pre-heated before processing so that the process temperature can reach and maintain the incipient denaturation temperature.

A typical method of practice is as follows:

A Model 2 1/2" Bematek Colloid Mill (Fig. 2; Bematek Systems, Beverly MA), was piped so that the inlet port was connected to a heat exchanger. Gas impermeable tubing was used to make the soft connections between the heat exchanger hose barbs.

The outlet port from the process head was connected to a stainless steel post-process chiller.

Solution temperature was monitored at three sites (T1, T2 and T3). The T1 thermocouple was mounted in a Swagelok "Tee" between the pre-heat heat exchanger and the mill head to measure the feed temperature of the protein solution. A second "Tee" for introducing gas was also placed at the feed port. The T2 thermocouple was placed inside the exit from the process head, approximately 1 cm from the rotor and 2 cm from the shaft so that the temperature of the process could be accurately measured. In this way, the two temperatures can be measured independently, the feed temperature (T1) and the process temperature, (T2), and compared to determine the amount of heating of the solution during processing.

For this example, U.S.P. albumin was diluted with normal saline to make up a 1% (w/v) solution. The denaturation temperature was determined experimentally, as described, to be 78°C. It was fed into the mill at 200 mL/min following degassing along with perfluoropropane at 100 mL/min (50% v/v). Differences between T1 and T2 of 10° to 15°C were noted. In order to obtain a process temperature of 77°C (1°C below denaturation temperature), the feed temperature was adjusted to a range of 62° to 67°C. Since the amount of heat generated will vary with different milling parameters, it is necessary to determine the difference between T1 and T2 with each change in milling parameters, (choice of mill, mill settings, flow rate, gas:liquid ratio, etc.) in order to target the process temperature to avoid bulk denaturation of the protein while successfully encapsulating the gas microbubbles with a thin shell of denatured protein.
The chiller-out temperature (T3) was also monitored, and for best results was targeted at 20°C.

### EXAMPLE 2

### Mechanical Cavitation Method of Making Microspheres Containing Different Gases

Microspheres containing various gases were produced as follows: 5% human albumin solution (USP) was deaerated under continuous vacuum for two hours. The vacuum was released by filling the evacuated vessel with the gas of interest. Insoluble gases utilized include sulfur hexafluoride, perfluoroethane, and perfluoropropane. Microspheres containing more soluble gases, air, nitrogen, oxygen and argon, were also produced. The use of argon was representative of a high molecular weight, but relatively soluble, gas. The albumin solution was adjusted to 68°C via an in-line heat exchanger and pumped at 100 mL/min into a 2 1/2" (63.5 mm) colloid mill (Greerco, Hudson, NH, model W250V or AF Gaulin, Everett, MA, model 2F). The specific gas, at room temperature, was added to the liquid feed just upstream of the inlet port at a flow rate of 120-220 mL/min. The gap between the rotor and the stator was adjusted to 2/1000th inch (0.005 cm) and the albumin solution was milled continuously at about 7000 rpm at a process temperature of 73°C.

The dense white solution of microspheres thus formed was immediately chilled to a temperature of 10°C by a heat exchanger, and collected in glass vials. The vials were immediately sealed. The material was characterized with regard to concentration and size distribution using a Coulter Counter. The results are shown in Table 3 below.

**Table 3**

| | **Concentration** **(µspheres/mL)** | **Mean Size** **(microns)** |
|---|---|---|
| Perfluoropropane | 8.3 x 10⁸ | 3.8 |
| Perfluoroethane | 10.6 x 10⁸ | 4.0 |
| Sulfur hexafluoride | 8.4 x 10⁸ | 3.9 |
| Air | 9.2 x 10⁷ | 3.4 |
| Nitrogen | 5.4 x 10⁷ | 5.0 |
| Oxygen | 6.1 x 10⁷ | 3.9 |
| Argon | 4.1 x 10⁷ | 3.5 |

### EXAMPLE 3

### Effect of Rotor Speed and Gap Size

A 1% albumin solution was combined (200 mL/min) with perfluoropropane (100 mL/min) at a 50% gas to liquid (v/v) ratio. Microspheres were prepared according to the procedure described in Example 1 using varying rotor speeds and gap sizes. The data obtained are shown in Table 4.

**Table 4**

| **Rotor Tip Speed** **(ft/min)** | **Gap (cm)** | **Concentration** **(µspheres/mL)** | **Mean Size** **(microns)** |
|---|---|---|---|
| 3500 | 0.01 | 0.76 x 10⁸ | 13.4 |
| 4300 | 0.01 | 2.43 x 10⁸ | 9.6 |
| 4800 | 0.01 | 9.38 x 10⁸ | 3.8 |
| 9700 | 0.01 | 20.96 x 10⁸ | 4.3 |
| 5200 | 0.02 | 12.87 x 10⁸ | 5.0 |
| 7000 | 0.02 | 12.50 x 10⁸ | 3.4 |
| 8700 | 0.02 | 14.42 x 10⁸ | 3.0 |
| 9600 | 0.02 | 15.22 x 10⁸ | 2.9 |

These results show that concentration increases and mean size decreases with increasing rotor speed while increasing gap size decreases concentration.

### EXAMPLE 4

### Effect of Gas to Liquid Ratio

A 0.5% Albumin solution (100 mL/min) was combined with perfluoropropane at 20, 50, 70 or 100 mL/min (20, 50, 70 or 100% gas to liquid v/v) using a Gaulin mill with an approximate gap of 0.012 and a rotor tip speed of 9950 ft/min (50.5 m/sec). The data obtained are shown in Table 5:

**Table 5**

| **Gas/Liquid % (v/v)** | **Gap** **(cm)** | **Concentration** **(µspheres/mL)** | **Mean Size** **(microns)** |
|---|---|---|---|
| 20 | 0.03 | 6.54 x 10⁸ | 3.6 |
| 50 | 0.03 | 7.51 x 10⁸ | 4.3 |
| 70 | 0.03 | 8.76 x 10⁸ | 5.0 |
| 100 | 0.03 | 8.66 x 10⁸ | 5.9 |

These results show that both concentration and mean size increase with an increase in gas:liquid ratio.

### EXAMPLE 5

### Microscopic Examination of Microspheres

Albumin microspheres containing various gases were prepared as described in Example 2. Microscopic examination of the products revealed a monodisperse suspension of spherical microspheres. The microspheres were collapsed by application of high pressure in a syringe until the suspension clarified. In all cases, microscopic reexamination revealed the presence of hyaline, membranous shells from the collapsed microspheres.

### EXAMPLE 6

### Pressure Resistance of Microspheres

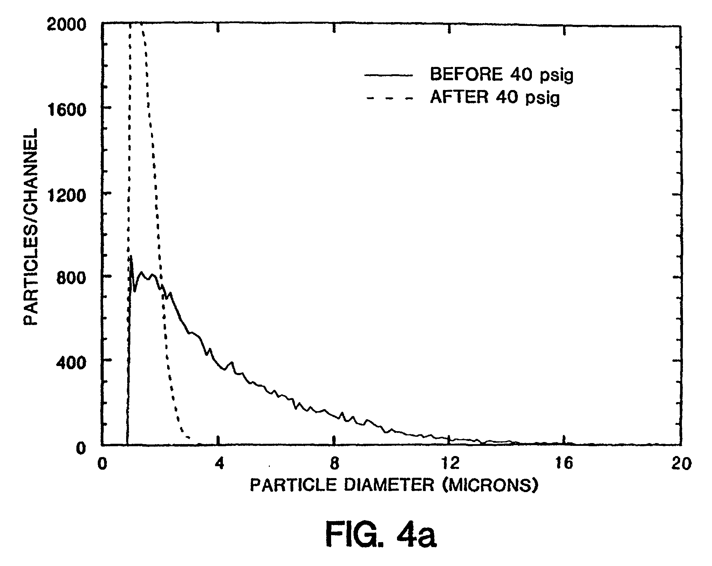

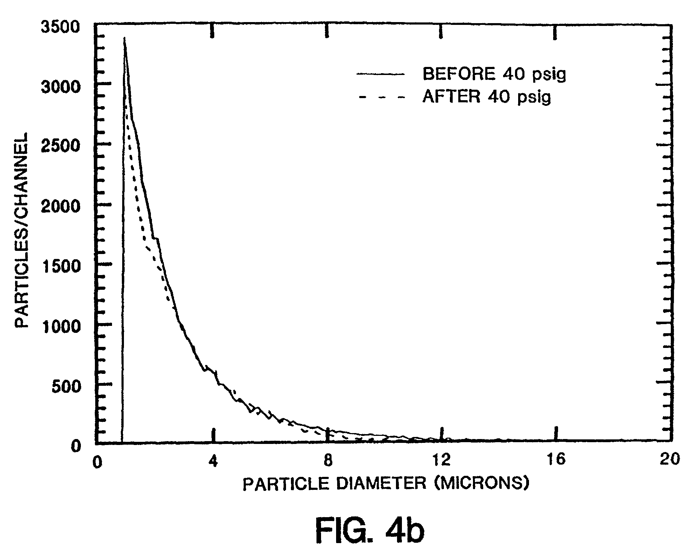

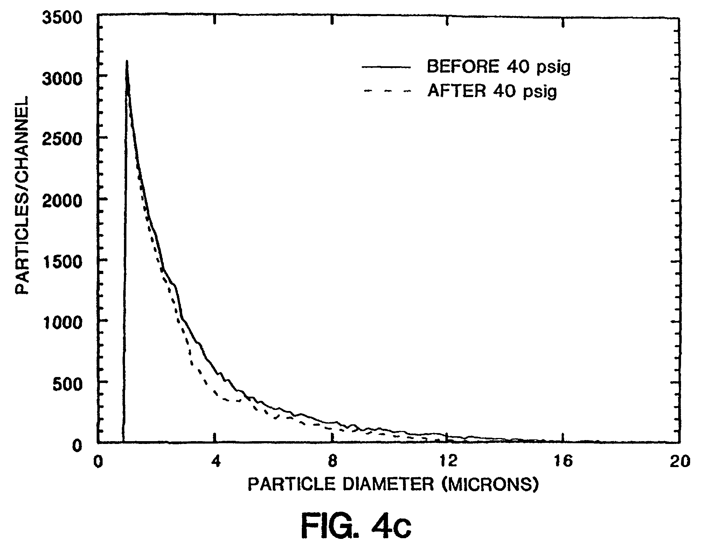

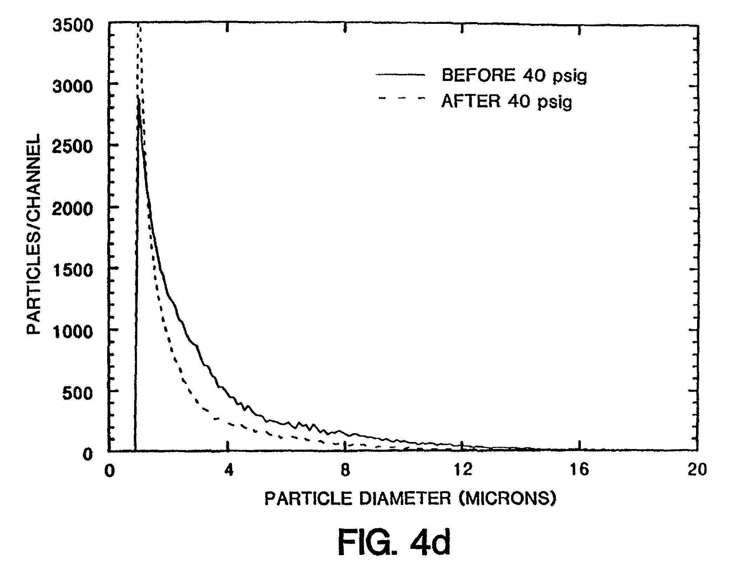

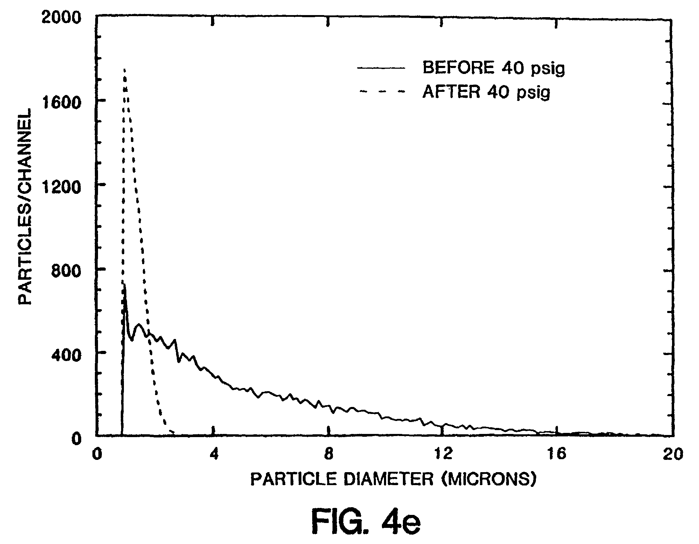

Albumin microspheres containing various gases were prepared as described in Example 2. Ten mL of each suspension was placed in a 10 mL glass gas-tight syringe (Hamilton, Reno NV) fitted with a pressure gauge. All headspace was removed and the apparatus was sealed. A constant pressure of 40 psig (2.76 bar) was applied for 3 min. A Coulter Counter was then used to measure the sample particle concentration and distribution. Comparisons of the data (Figures 4a-4e) before and after pressurization demonstrated a relative resistance of the insoluble gas microspheres to 40 psig (2.76 bar).

### EXAMPLE 7

### Pressure Resistance of Dilute Suspensions of Microspheres

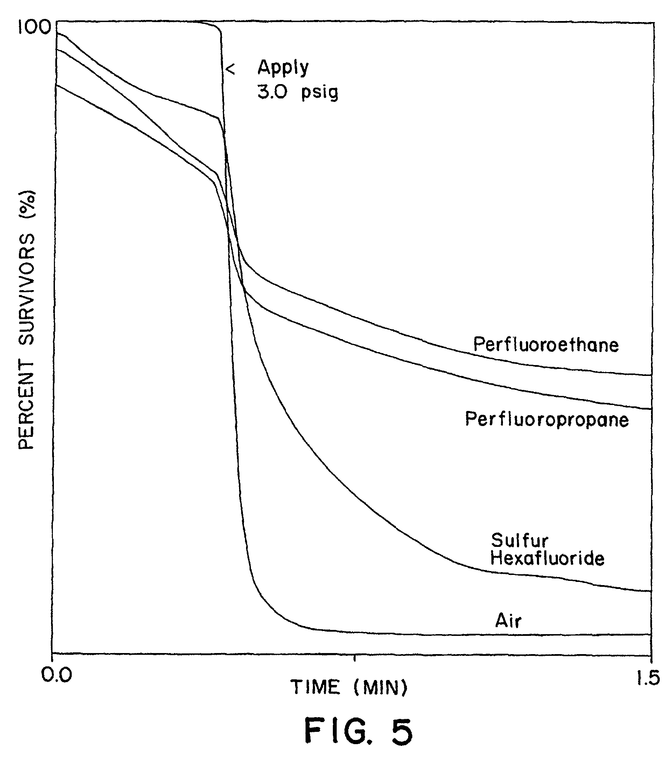

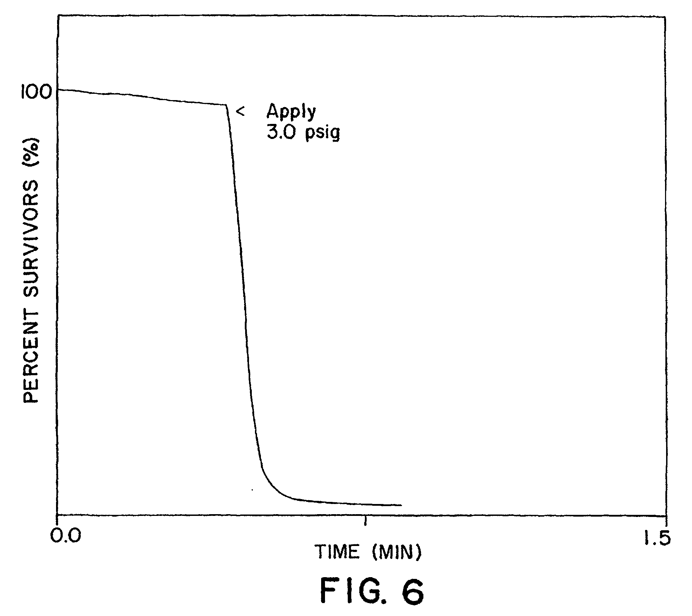

Microspheres containing various gases were prepared as described in Example 2. Each sample of microspheres was diluted to an equal volume of encapsulated gas per mL of phosphate-buffered saline (0.15M), about a 1:60 dilution. The diluted suspension was subjected to instant static pressures of 0.5 psig (0.035 bar) to 7.5 psig (0.52 bar) in a sealed vessel with adequate head space. Figure 5 shows the effect of pressure on microsphere concentration. Microspheres containing the insoluble gases perfluoropropane, perfluoroethane and sulfur hexafluoride are much more pressure-resistant than air or high molecular weight argon-filled microspheres of the same concentration and size distributions (Figure 6). Physiological pressures in the bloodstream range from a peripheral venous pressure of 1.5 psig (0.10 bar) to 2.5 psig (0.17 bar) in the myocardial wall.

### EXAMPLE 8

### Effect of Degassed Buffer on Microspheres

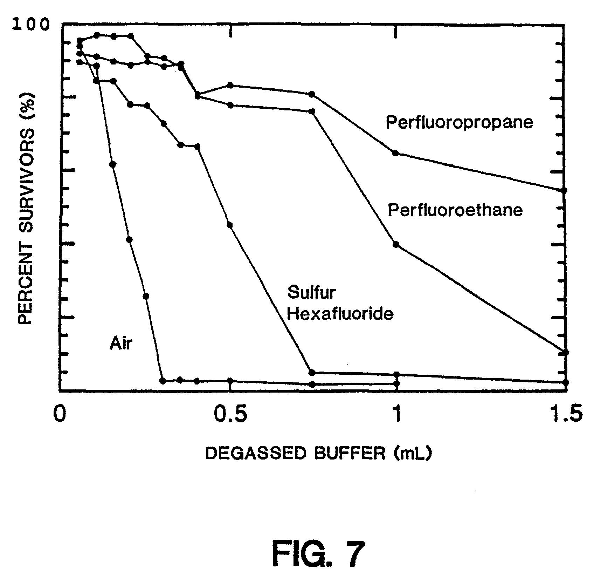

Albumin microspheres containing various gases were prepared as described in Example 2. Phosphate-buffered saline (PBS) was degassed by boiling just before use. 0.05 mL to 1.5 mL aliquots of the hot buffer were placed in 13 x 100 test tubes and allowed to cool 1 min to room temperature in a water bath. A constant volume of microspheres was added to each tube. After mixing, the final volume was brought to 3.0 mL with PBS and the microsphere concentration was determined. Figure 7 shows that improved survival in degassed solutions is obtained for the microspheres containing the insoluble gases perfluoropropane, perfluoroethane and sulfur hexafluoride.

Microspheres containing air, sulfur hexafluoride or perfluoroethane were diluted into whole blood. Air-filled microspheres exhibited collapse. The insoluble gas-filled microspheres were shown to survive dilution in fresh whole blood.

### EXAMPLE 9

### Elasticity

Microspheres prepared from various gases were prepared as described in Example 2. Microspheres were diluted into phosphate-buffered saline, as described in Example 7, and placed in a clear cell positioned on the stage of a microscope. The cell was connected to a nitrogen source that allowed for observing the effects of rapid application and release of physiological pressures on the microspheres.

The application of 1.5 psig (0.10 bar) or greater to the soluble gas containing microspheres resulted in observing the complete loss of spherical bodies. The microspheres did not reform upon the release of the pressure, indicating irreversible destruction. The application of less than 1.5 psig (0.10 bar) resulted in deformation and wrinkling of the shell with incomplete loss of microspheres. The spherical appearance or population could not be restored upon release of the applied pressure.

The application of pressure up to several psig to a suspension of microspheres containing the insoluble perfluorocarbon gases resulted in a reduction of the diameter of the microspheres. The diameter of the microspheres returned to the original dimensions upon the release of the pressure.

Sulfur hexafluoride microspheres also exhibited enhanced elasticity under applied physiological pressure relative to air-filled microspheres, but less elasticity relative to the perfluorocarbon microspheres.

These observations indicate that the microspheres containing insoluble gases were not only resistant to pressure, but recovered after pressure was released. This is indicative of an elastic protein shell.

### EXAMPLE 10

### Comparison of Microspheres Made in an Open System and in a Closed System

### PROCESS METHODS

### A) Manual Sonication: Open System (Equivalent to EPA 554,213 One-Step Method)

The method described in U.S. Patent No. 4,844,882 and European Patent Application 554,213 was used to prepare microspheres as follows:

A 20 cc syringe barrel was fitted with a T-type thermocouple inserted through the tip and mounted onto a support stand. The syringe was filled to the 16 cc mark with Swiss Red Cross 5% human serum albumin. Gas (perfluoropropane (C₃F₈) or sulfur hexafluoride (SF₆)) was introduced into the top of the syringe barrel and flowed over the surface of the liquid. A sonicating horn was lowered to the 10 cc mark, below the surface of the solution, and operated at 50% power until the temperature of the solution rose to 72.8 - 73°C; approximately 1 minute. The horn was immediately withdrawn to the meniscus ± 1 mm and the power level increased to 65%. Sonication continued for 5 seconds, with an additional temperature increase of 1.2 - 2°C. The product was poured into a glass vial to capacity and sealed.

### B) Continuous Sonication: Closed System

The method described in U.S. Patent No. 4,957,656 was used to prepare perfluoropropane and sulfur hexafluoride microspheres as follows:

Human serum albumin was diluted to a 1% w/v solution with sterile saline. The solution was heated to incipient denaturation, approximately 76°C. The system was closed to the external atmosphere and perfluoropropane or sulfur hexafluoride gas was introduced into the liquid flow (1:1) in place of air. The product was made continuously by flowing the gas/albumin mixture past the sonicator horn at approximately 100 ml liquid/min. The product was chilled upon exit from the sonication chamber by passage through a heat exchanger and collected as a bulk liquid suspension of microspheres. Handling and storage conditions were similar to that given for manually produced microspheres.

### C) Mechanical Cavitation: Closed System

Albumin microspheres containing perfluoropropane or sulfur hexafluoride gas were also produced in a closed system by milling a mixture of 1% human serum albumin and gas, similar to that described in Example 2. Albumin solution, heated to a temperature sufficient to allow microsphere formation by the mechanical cavitation of a given mill, was mixed 1:1 (v:v) gas and introduced into a colloid mill. The liquid flow rate was dependent upon the capacity or size of the mill, typically 100 to 500 ml/min. A Silverson L4R mill and a Bematek 3" (76.2 mm) production colloid mill were used for this evaluation. The outflow from the mill was cooled by passage through a heat exchange system and the resulting albumin microsphere suspension was collected in bulk. The product was filled into glass vials, similar to the other processes.

### ANALYTICAL METHODS

### A) Population Dynamics

Population dynamics were evaluated with a Coulter Multisizer II using a 50 micron aperture. Albumin microspheres prepared as described in PROCESS METHODS were diluted 1:10,000 into Isoton and a 500 µl sample was analyzed. Concentration, mean size, and encapsulated gas volume per ml of the original microsphere suspension were obtained.

### B) Gas Content

The percentage of perfluoropropane entrapped in duplicate lots of microspheres prepared as described in PROCESS METHODS was determined by gas chromatography on a Hewlett Packard 5890. A sample of the microsphere suspension was taken in a gas tight syringe. The gas was released from the microspheres using an anti-foam agent in ethanol and the entrapped gas was detected by thermal conductivity.

### C) Pressure Resistance

Pressure resistance of albumin microspheres was evaluated by a method similar to that reported by Sintetica in European Patent Application 554,213. Microspheres were diluted in aerated phosphate buffered saline, to approximately 1 absorbance unit at 600 nm, in a 3 ml pressure cuvette. The neck was attached to a pressure source and the cuvette placed in a recording spectrophotometer. The pressure in the cuvette was increased linearly from 0 to 5 or 10 psig (0.35 to 0.69 bar) over 150 seconds, at which time the pressure was released. The pressure ramp was created by a proportioning solenoid valve (Honeywell) and a pressure transducer (Omega) that were placed between a 20 psi (1.38 bar) pressure source (N₂ tank) and a 5 liter stainless steel reservoir. The cuvette was connected to the steel reservoir through a digital pressure gauge. A PC-type computer equipped with an analog to digital converter and a digital to analog converter board (National Instruments) controlled the opening of the valve and read the pressure transducer. The reservoir, and cuvette, was pressurized at a selected rate until the desired pressure was achieved. The optical density of the microsphere suspension was monitored as a function of time and pressure. The data was corrected for the natural flotation rate of microspheres in the cuvette.

### RESULTS

### A) Population Dynamics

Albumin microspheres produced by the methods of manual sonication, continuous sonication and mechanical cavitation were analyzed for concentration, mean size, encapsulated gas volume and size distribution within 24 hours after manufacture. All measurements were performed in duplicate, as a minimum, and are presented as the average. The results of these measurements are given in Table 6.

**Table 6**

| **Gas** | **Method** | **Conc.** **(10**^{**8**}**/ml)** | **Mean Size** **(**µ**m)** | **Vol** **(ml/ml)** |
|---|---|---|---|---|
| SF₆ | Manual Sonication | 8.7 | 3.2 | 0.046 |
| SF₆ | Continuous Sonication | 12.7 | 2.7 | 0.034 |
| SF₆ | Mechanical Cavitation | 10.0 | 3.8 | 0.054 |
| C₃F₈ | Manual Sonication | 13.4 | 2.8 | 0.033 |
| C₃F₈ | Manual Sonication | 17.7 | 2.8 | 0.056 |
| C₃F₈ | Continuous Sonication | 10.1 | 3.0 | 0.050 |
| C₃F₈ | Continuous Sonication | 6.7 | 4.3 | 0.127 |
| C₃F₈ | Mechanical Cavitation (Bematek Mill) | 31.0 | 3.0 | 0.23 |
| C₃F₈ | Mechanical Cavitation (Silverson Mill) | 6.9 | 5.0 | 0.34 |

Microspheres produced by all methods were stable for the duration of this study, at least several weeks at 4°C.

### B) Gas Content

Analyses of the composition of entrapped perfluoropropane gas in duplicate lots of microspheres are given in Table 7.

**Table 7**

| **Method******* | **%C**_{**3**}**F**_{**8**} |
|---|---|
| Manual Sonication | 70.0 |
| Continuous Sonication | 89.5 |
| Mechanical Cavitation | 95.5 |

| | |
|---|---|
| * Average results of duplicate lots. | |

These results demonstrate that microspheres made in the open system using manual sonication encapsulate much less of the gas used to form the microspheres than those made in the closed systems (continuous sonication and mechanical cavitation.) The microspheres made in the closed system were made in the absence of oxygen, as determined using an oxygen electrode. Microspheres made by all three methods were subjected to the same amount of exposure to the atmosphere during handling and sampling (which accounts for less than 100% perfluoropropane gas being measured in microspheres made using the two closed system procedures), thus there was oxygen (and other atmospheric gases) present during formation in the open system which diminished the efficiency of gas encapsulation.

### C) Pressure Resistance

A suspension of gas-filled microspheres will decrease in optical density with increasing pressure due to a decrease in size and associated change in surface area. Shrinkage is due to two factors; reversible compression according to the gas laws, and irreversible loss of the gas core to the surrounding liquid due to increased solubility according to Henry's law. Upon the release of an applied pressure, only that fraction of the volume loss due to compression is recovered, and which can be observed by an increase in optical density. The loss of entrapped gas to the surrounding solution does not reenter the microspheres upon depressurization, but is lost to the head space above the solution.

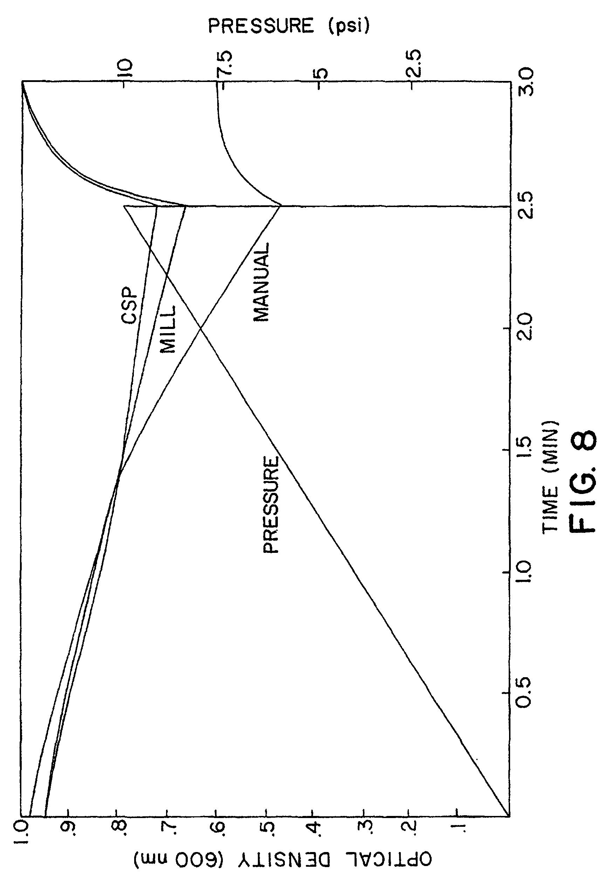

Fig. 8 shows the result of imposing a linear pressure gradient up to 10 psi (0.69 bar) on 1 OD suspensions of albumin microspheres prepared with perfluoropropane gas by the manual sonication (open system) method as well as the continuous sonication and mechanical cavitation (closed system) methods. Both closed system methods yielded microspheres that exhibited compression with increasing pressure, with a total recovery of volume upon release of the pressure at the end of the gradient. Loss of entrapped gas to the surrounding solution was not observed. Albumin microspheres prepared in the open system (manual sonication method) exhibited greater compression with applied pressure and only a partial recovery of volume upon release of pressure due to the irreversible loss of the gas core, resulting in a 40% destruction of microspheres.

## Claims

1. A method of making encapsulated gas microspheres useful as an ultrasonic imaging agent the method comprising:
a. providing an aqueous solution of a heat-denaturable protein at a temperature necessary to achieve incipient denaturation temperature during subsequent mechanical emulsification;
b. combining the solution with a gas;
c. emulsifying the protein solution and gas mixture by mechanically shearing the mixture to form a suspension of gas microbubbles having a mean diameter in the range of about 0.1 to about 10 microns; and
d. encapsulating the gas microbubbles to form microspheres by mechanically cavitating the suspension to cause the protein to become denatured and thereby deposited at the gas-solution interface.

2. A method according to claim 1 wherein the temperature is achieved by heating the solution.

3. A method according to claim 1 wherein the temperature is achieved by including additives in the solution that alter the denaturation temperature of the protein.

4. A method according to any preceding claim wherein the protein is a naturally occurring protein.

5. A method according to any preceding claim wherein the protein is human serum albumin.

6. A method according to any of claims 1 to 3 wherein the protein is a synthetic protein.

7. A method according to any preceding claim wherein the concentration of the protein in the solution is from 0.1 to 10% w/v.

8. A method according to any preceding claim wherein the concentration of the protein in the solution is from 1 to 5% w/v.

9. A method according to any preceding claim wherein the concentration of the protein in the solution is about 1% w/v.

10. A method according to any preceding claim wherein the gas is insoluble.

11. A method according to any preceding claim wherein the gas is sulphur hexafluoride, perfluoromethane, perfluoroethane, perfluoropropane or perfluorobutane.

12. A method according to any of claims 1 to 9 wherein the gas is air.

13. A method according to any preceding claim wherein the ratio of gas to protein solution is 5% to 200% v/v.

14. A method according to any preceding claim wherein the ratio of gas to protein solution is 20% to 100% v/v.

15. A method according to any preceding claim wherein steps (c) and (d) are effected by passing the mixture through a mill.

16. A method according to any preceding claim wherein the incipient denaturation temperature is about 1°C to 5°C below the denaturation temperature of the protein.

## Patentansprüche

1. Verfahren zur Herstellung verkapselter gasenthaltender Mikrokügelchen, welche als ein bildgebendes Mittel für Ultraschall verwendbar sind, wobei das Verfahren die Schritte umfasst:
a) Bereitstellen einer wässrigen Lösung eines wärmedenaturierbaren Proteins bei einer Temperatur, die erforderlich ist, um die Temperatur des Beginns der Denaturierung während nachfolgender mechanischer Emulgierung zu erreichen;
b) Vereinigung der Lösung mit einem Gas;
c) Emulgieren des Proteinlösung-/Gas-Gemischs durch mechanische Scherung des Gemischs, um eine Suspension von gasenthaltenden Mikrobläschen mit einem mittleren Durchmesser im Bereich von etwa 0,1 bis etwa 10 µm zu bilden; und
d) Verkapseln der gasenthaltenden Mikrobläschen unter Bildung von Mikrokügelchen durch mechanische Hohlraumbildung in der Suspension, wodurch das Protein denaturiert und dadurch an der Gas-Lösungsgrenzfläche abgeschieden wird.

2. Verfahren gemäß Anspruch 1, wobei die Temperatur durch Erwärmen der Lösung erreicht wird.

3. Verfahren gemäß Anspruch 1, wobei die Temperatur durch Verwendung von Zusatzstoffen in der Lösung, welche die Denaturierungstemperatur des Proteins verändern, erreicht wird.

4. Verfahren gemäß einem der vorhergehenden Ansprüche, wobei das Protein ein natürlich vorkommendes Protein ist.

5. Verfahren gemäß einem der vorhergehenden Ansprüche, wobei das Protein Humanserumalbumin ist.

6. Verfahren gemäß einem der Ansprüche 1 bis 3, wobei das Protein ein synthetisches Protein ist.

7. Verfahren gemäß einem der vorhergehenden Ansprüche, wobei die Konzentration des Proteins in der Lösung von 0,1 bis 10 % w/v ist.

8. Verfahren gemäß einem der vorhergehenden Ansprüche, wobei die Konzentration des Proteins in der Lösung von 1 bis 5 % w/v ist.

9. Verfahren gemäß einem der vorhergehenden Ansprüche, wobei die Konzentration des Proteins in der Lösung etwa 1 % w/v ist.

10. Verfahren gemäß einem der vorhergehenden Ansprüche, wobei das Gas unlöslich ist.

11. Verfahren gemäß einem der vorhergehenden Ansprüche, wobei das Gas Schwefelhexafluorid, Perfluormethan, Perfluorethan, Perfluorpropan oder Perfluorbutan ist.

12. Verfahren gemäß einem der Ansprüche 1 bis 9, wobei das Gas Luft ist.

13. Verfahren gemäß einem der vorhergehenden Ansprüche, wobei das Verhältnis von Gas zu Proteinlösung 5 % bis 200 % v/v ist.

14. Verfahren gemäß einem der vorhergehenden Ansprüche, wobei das Verhältnis von Gas zu Proteinlösung 20 % bis 100 % v/v ist.

15. Verfahren gemäß einem der vorhergehenden Ansprüche, wobei die Schritte (c) und (d) durch Durchleiten des Gemischs durch eine Mühle bewirkt werden.

16. Verfahren gemäß einem der vorhergehenden Ansprüche, wobei die Temperatur des Beginns der Denaturierung etwa 1 °C bis 5 °C unterhalb der Denaturierungstemperatur des Proteins liegt.

## Revendications

1. Procédé de fabrication de microsphères de gaz encapsulé utiles comme agent d'imagerie ultrasonique, le procédé comprenant les opérations consistant à :
a. prendre une solution aqueuse d'une protéine pouvant être dénaturée thermiquement à une température nécessaire pour obtenir une température de début de dénaturation pendant l'émulsification mécanique subséquente ;
b. combiner la solution avec un gaz ;
c. émulsifier la solution de protéine et le mélange gazeux en cisaillant mécaniquement le mélange pour former une suspension de microbulles de gaz ayant un diamètre moyen dans la gamme d'environ 0,1 à environ 10 micromètres ; et
d. encapsuler les microbulles de gaz pour former des microsphères par cavitation mécanique de la suspension pour provoquer la dénaturation de la protéine et la déposer par ce moyen à l'interface gaz-solution.

2. Procédé selon la revendication 1 dans lequel la température est obtenue par chauffage de la solution.

3. Procédé selon la revendication 1 dans lequel la température est obtenue en incluant des additifs dans la solution qui modifient la température de dénaturation de la protéine.

4. Procédé selon une revendication précédente quelconque dans lequel la protéine est une protéine naturelle.

5. Procédé selon une revendication précédente quelconque dans lequel la protéine est l'albumine sérique humaine.

6. Procédé selon l'une quelconque des revendications 1 à 3 dans lequel la protéine est une protéine synthétique.

7. Procédé selon une revendication précédente quelconque dans lequel la concentration de la protéine dans la solution varie de 0,1 à 10 % w/v.

8. Procédé selon une revendication précédente quelconque dans lequel la concentration de la protéine dans la solution varie de 1 à 5 % w/v.

9. Procédé selon une revendication précédente quelconque dans lequel la concentration de la protéine dans la solution est d'environ 1 % w/v.

10. Procédé selon une revendication précédente quelconque dans lequel le gaz est insoluble.

11. Procédé selon une revendication précédente quelconque dans lequel le gaz est l'hexafluorure de soufre, le perfluorométhane, le perfluoroéthane, le perfluoropropane ou le perfluorobutane.

12. Procédé selon l'une quelconque des revendications 1 à 9 dans lequel le gaz est l'air.

13. Procédé selon une revendication précédente quelconque dans lequel le rapport de gaz à la solution de protéine est de 5 % à 200 % v/v.

14. Procédé selon une revendication précédente quelconque dans lequel le rapport de gaz à la solution de protéine est de 20 % à 100 % v/v.

15. Procédé selon une revendication précédente quelconque dans lequel dans lequel les étapes (c) et (d) sont effectuées en faisant passer le mélange à travers un broyeur.

16. Procédé selon une revendication précédente quelconque dans lequel la température de début de dénaturation est d'environ 1°C à 5°C au-dessous de la température de dénaturation de la protéine.
